# EUROPÄISCHE PATENTANMELDUNG

(11) **EP 3 159 039 A1**
(43) Veröffentlichungstag der Anmeldung: **26.04.2017**
(21) Anmeldenummer: 16194749.4
(22) Anmeldetag: 20.10.2016
(51) Int. Cl.: A61N 1/375

(54) **DURCHFÜHRUNG EINES MEDIZINELEKTRONISCHEN GERÄTES UND MEDIZINELEKTRONISCHES GERÄT**

(30) Priorität: 21.10.2015 DE 102015117935
(71) Anmelder: BIOTRONIK SE & Co. KG, 12359 Berlin (DE)
(72) Erfinder: Sontheimer, Thomas, 90574 Roßtal (DE)
(74) Vertreter: Galander, Marcus

(57) **Zusammenfassung**

Durchführung eines, insbesondere implantierbaren, medizinelektronischen Gerätes, mit einem Gehäuse und mindestens einem in dem Gehäuse aufgenommenen elektrischen oder elektronischen Bauelement, wobei die Durchführung einen Durchführungs-Flansch zum Verschluss einer Öffnung des Gehäuses und zur Halterung mindestens eines Anschlusselementes das zum gehäuse-externen Anschluss des oder mindestens eines Bauelementes dient, in einem das Anschlusselement umgebenden Isolierelement aufweist und ein Massepin in mechanischem und elektrischem Kontakt mit dem Durchführungs-Flansch zur Realisierung eines Masseanschlusses des Gerätes vorgesehen ist, wobei der Massepin ausschließlich durch Form- und Kraftschluss in dem Durchführungs-Flansch oder dem Isolierelement, unter Berührung des Durchführungs-Flansches, fixiert ist.

## Beschreibung

Die Erfindung betrifft eine Durchführung eines, insbesondere implantierbaren, medizinelektronischen Gerätes mit einem Gehäuse und mindestens einem in dem Gehäuse aufgenommenen elektrischen oder elektronischen Bauelement, wobei die Durchführung einen Durchführungs-Flansch zum Verschluss einer Öffnung des Gehäuses und zur Halterung mindestens eines Anschlusselementes, das zum gehäuse-externen Anschluss des oder mindestens eines Bauelementes dient, in einem das Anschlusselement umgebenden Isolierelement aufweist und ein Massepin in mechanischem und elektrischem Kontakt mit dem Durchführungs-Flansch zur Realisierung eines Masseanschlusses des Gerätes vorgesehen ist. Sie betrifft des Weiteren ein Verfahren zur Herstellung einer solchen Durchführung sowie ein medizinelektronisches Gerät, das eine derartige Durchführung aufweist.

Implantierbare Geräte der oben bezeichneten Art sind insbesondere als Herzschrittmacher oder implantierbare Kardioverter (speziell Defibrillatoren) seit langem in massenhaftem Einsatz. Es kann sich hierbei aber auch um eine weniger komplexe Vorrichtung, wie etwa eine Elektroden- oder Sensorleitung, handeln. Neben dem Einsatz von Durchführungen in Geräten für die Herztherapie finden Durchführungen auch in Cochlearimplantaten Anwendung.

Die meisten praktisch bedeutsamen implantierbaren elektromedizinischen Geräte sind dazu vorgesehen, über geeignet platzierte Elektroden elektrische Impulse an reizbares Körpergewebe abzugeben. Um diese Funktion auszuführen, sind im Gehäuse des Gerätes elektronische/elektrische Funktionseinheiten zur Erzeugung der Impulse und zur geeigneten Steuerung der Impulserzeugung untergebracht, und außen am Gerät sind unmittelbar Elektro-den oder Anschlüsse für mindestens eine Elektrodenleitung vorgesehen, in deren distalem Endabschnitt die Elektroden zur Impulsübertragung an das Gewebe angebracht sind. Die elektronischen/elektrischen Funktionseinheiten im Geräteinneren sind in einer Weise mit den äußeren Elektroden oder Elektrodenleitungsanschlüssen zu verbinden, die unter den speziellen Bedingungen des implantierten Zustandes eine absolut und dauerhaft verlässliche Funktion gewährleistet. Die Anschlüsse oder Elektrodenleitungen können auch genutzt werden, um elektrische Impulse und Reize im Körper des Patienten gezielt zu messen und über einen längeren Zeitraum aufzuzeichnen oder auszuwerten, um eine individuell angepasste Therapie zu wählen und den Erfolg der Behandlung zu überprüfen.

Bekannt sind insbesondere Durchführungen, deren Grund- und Isolationskörper im Wesentlichen aus Keramik oder Glas besteht, wobei auch mehrschichtige bzw. mehrteilige Aufbauten unter Einsatz von Metallen oder Metalloxiden entwickelt wurden und eingesetzt werden. Derartige bekannte Durchführungen erfüllen weitgehend die an sie gestellten Anforderungen von Hermetizität, Biokompatibilität, Signalübertragung, und Langzeitstabilität.

Zur Bereitstellung eines Massepotentials für die elektronischen/elektrischen Bauelemente und Baugruppen des Gerätes wird eine Verbindung zu dessen metallischem Gehäuse hergestellt, und zwar typischerweise durch ein spezielles Masse-Verbindungsmittel im Bereich der Durchführung, speziell einen sogenannten Massestift (Massepin). Ein Massestift, beispielsweise aus Niob oder Pt/Ir, wird bei einigen Typen bekannter Geräte mittels Widerstandsschweißen am Gehäuse aus Titan gefügt. Die elektrische Verbindung zwischen Gehäuse und Platine entsteht nach dem Schweißen oder Löten des Massepins auf der Platine und dem Einschweißen des Flansches mit dem Gehäuse. Alternativ erfolgt die Masseanbindung mittels eines Stiftes, welcher während des Bestückens in eine Sacklochbohrung montiert und im Hochtemperaturlötverfahren mit dem Flansch verlötet wird. Die elektrische Verbindung durch das Gehäuse entsteht nach dem Weichlöten der Durchführung auf der Platine und dem Verschweißen des Flansches mit dem Gehäuse.

Der aktuelle Schweißprozess des Massepins ist im Hinblick auf seine Prozessstabilität weniger befriedigend. Aufgrund der unterschiedlichen Schmelzpunkte der Fügepartner Nb und Ti ist ein Anheften des Nb-Stiftes an den Ti-Flansch mittels Schweißens eher ungeeignet. Das aktuelle Herstellen des Massekontakts in bestimmten Varianten der Durchführung mittels HTL-Prozess benötigt Bestückungszeit und zusätzliches Lotmaterial in Form von Gold. Dadurch, dass der Massestift in den Metallflansch zusammen mit der eigentlichen Verlötung (von Pins in die Isolationskeramik und der Keramik in den Flansch) verlötet werden muss, ist das Prozessfenster für den Lötprozess stark eingegrenzt und ein Optimieren des Lötprofils für das eigentliche Einlöten der Keramik wird erschwert. Es ist feststellbar, dass der Massepin meist bauartbedingt die heißeste Stelle beim Verlöten ist, was zu Sekundäreffekten führt und sich bei manchen Durchführungstypen in Form von Metallabdampfungen wiederspiegelt. Dies führt zu unnötigen, nachgeschalteten Reinigungsprozessen.

Der Erfindung liegt die Aufgabe zugrunde, eine verbesserte Durchführung eines implantierbaren elektromedizinischen Gerätes bereitzustellen, bei der insbesondere die materialseitigen Probleme der bekannten Schweiß- und Lötverfahren vermieden werden sollen und hierdurch der Herstellungsprozess und die hierin gebildete Durchführung weniger störanfällig sein soll. Zugleich soll der Herstellungsaufwand möglichst gering gehalten werden. Des Weiteren soll ein geeignetes Verfahren zur Herstellung einer solchen Durchführung sowie ein verbessertes implantierbares medizinelektronisches Gerät angegeben werden.

Diese Aufgabe wird in einem ersten Vorrichtungsaspekt durch eine Durchführung mit den Merkmalen des Anspruchs 1 und gemäß einem zweiten Vorrichtungsaspekt durch ein Gerät mit den Merkmalen des Anspruchs 13 sowie in ihrem Verfahrensaspekt durch ein Verfahren mit den Merkmalen des Anspruchs 8 gelöst. Zweckmäßige Fortbildungen des Erfindungsgedankens sind Gegenstand der jeweiligen abhängigen Ansprüche.

Die Erfindung schließt den Gedanken einer bewussten Abwendung von einer stoffschlüssigen Verbindung des Massepins mit dem umgebenden bzw. räumlich zugeordneten Durchführungs-Teil ein. Sie schließt weiter den Gedanken ein, diese stoffschlüssige Verbindung durch eine Kombination aus form- und kraftschlüssiger Verbindung zu ersetzen. Insgesamt führt das zu der Lehre, dass der Massepin ausschließlich durch Form- und Kraftschluss in dem Durchführungs-Flansch oder dem Isolierelement, unter Berührung des Durchführungs-Flansches, fixiert ist.

Durch das direkte Einbringen des Massestifts in den Flansch während der Flanschherstellung, fallen zusätzliche Fügeprozesse in Form von Massestiftschweißen oder zusätzliche Bestückungsaufwände (beim HT-Löten) und zusätzliche Materialbedarfe weg. Des Weiteren kann, bezogen auf die eingangs erwähnte spezielle Prozessführung, ein Optimieren des Lötprofils auf die Fügepartner Keramik und Metall, ohne den Hotspot Massepin stattfinden, was eine erhöhte Prozessstabilität des HTL-Prozesses zur Folge hat und helfen kann, ursächlich Sekundärfehler wie Abdampfungen zu verhindern. Dies wiederum könnte Nachbearbeitungsschritte obsolet werden lassen, die kostenintensiv sind und wiederum zu anderen, ungewünschten Sekundäreffekten führen.

In einer Ausführung der Erfindung ist das Isolierelement als massiver keramischer Isolierkörper ausgebildet und der Massepin in den Isolierkörper eingesintert. Verfahrensseitig stellt sich diese Ausführung so dar, dass das Isolierelement eine an die Gestalt des Massepins angepasste Ausnehmung aufweist, der Massepin im "grünen" Zustand des ebenfalls "grünen" Isolierkörpers in diesen eingefügt wird und der Isolierkörper mit eingefügtem Massepin in einem Sinterverfahren, bevorzugt zusammen mit dem "grünen" Flansch, fertiggestellt wird.

Eine weitere Ausführung zeichnet sich dadurch aus, dass der Durchführungs-Flansch oder das Isolierelement ein Kunststoff-Spritzgussteil aufweist und der Massepin mit dem Kunststoff-Spritzgussteil umspritzt ist. Diese Ausführung wird verfahrensseitig so realisiert, dass der Massepin in eine Spritzgussform zur Bildung des Durchführungs-Flansches oder Isolierelementes eingelegt und mit einem in die Form eingebrachten Kunststoffmaterial umspritzt wird.

In einer noch weiteren Ausführung ist vorgesehen, dass der Durchführungs-Flansch einen Flanschkörper aus einem ersten Material aufweist und der Massepin aus einem zweiten Material gebildet ist, das einen kleineren thermischen Ausdehnungskoeffizienten als das erste Material aufweist, und der Massepin in den Flanschkörper eingeschrumpft ist. In einer Ausgestaltung ist der Flanschkörper ein Metallkörper aus einem ersten Metall, insbesondere Titan, und der Massepin besteht aus einem zweiten Metall, das einen kleineren thermischen Ausdehnungskoeffizienten als das erste Metall hat, insbesondere aus Niob. Diese Ausgestaltung wird verfahrenstechnisch so realisiert, dass der Massepin in eine Metallspritzform zur Bildung des Durchführungs-Flansches eingelegt und anschließend die Form mit einem verflüssigten Metall gefüllt und hierbei der Massepin mit dem Metall umspritzt wird.

In einer anderen Ausgestaltung der vorab erwähnten Ausführung ist der Massepin durch thermisches Aufschrumpfen des Flanschkörpers auf den Massepin in den Flanschkörper eingebettet. Das zugehörige Herstellungsverfahren sieht vor, dass ein vorgefertigter Durchführungs-Flansch aus dem ersten Metall, in dem eine Ausnehmung zur Aufnahme des Massepins gebildet ist, erwärmt und im erwärmten Zustand der Massepin in die Ausnehmung eingebracht wird. Anschließend wird der Durchführungs-Flansch mit eingebrachtem Massepin abgekühlt, derart, dass der Massepin in den Durchführung-Flansch eingeschrumpft wird.

Vorteile und Zweckmäßigkeiten der Erfindung ergeben sich im Übrigen aus der nachfolgenden Beschreibung eines Ausführungsbeispiels anhand der Figuren. Von diesen zeigen:
- Fig. 1: eine schematische, teilweise geschnittene Darstellung eines implantierbaren elektromedizinischen Geräts,
- Fig. 2A und 2B: Prinzipskizzen von Durchführungen (Draufsicht) zur Erläuterung von Ausführungsbeispielen der Erfindung.

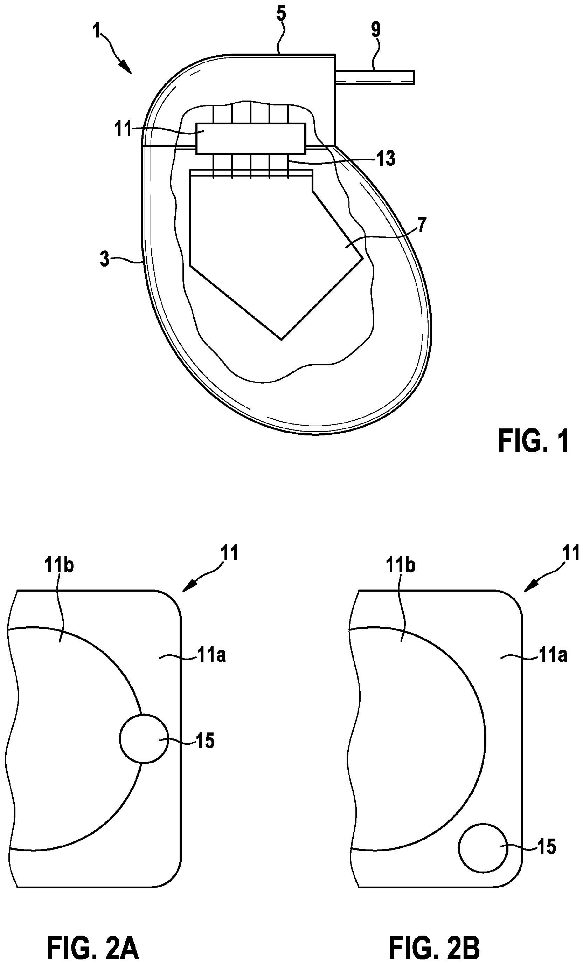

Fig. 1 zeigt einen Herzschrittmacher 1 mit einem Schrittmachergehäuse 3 und einem Kopfteil (Header) 5, in dessen Inneren neben anderen elektronischen Komponenten eine Leiterplatte (PCB) 7 angeordnet und mit dessen im Header angeordneten (nicht gezeigten) Leitungsanschluss eine Elektrodenleitung 9 verbunden ist. Eine zwischen Gerätegehäuse 3 und Header 5 vorgesehene Durchführung 11 umfasst eine Mehrzahl von Anschlussstiften 13. Die Anschlussstifte sind an einem Ende durch eine entsprechende Bohrung in die Leiterplatte gesteckt und mit dieser weich verlötet.

Fig. 2A und 2B zeigen Prinzpskizzen (Draufsichten) zweier leicht voneinander abweichender Durchführungen 11 zur Erläuterung von Ausführungsbeispielen der Erfindung. Bei der Konfiguration nach Fig. 2A umfasst die Durchführung 11 einen Durchführungs-Flansch 11a und einen innerhalb des Flansches angeordneten massiven Isolierkörper 11b, und in beide Komponenten ist eine annähernd halbkreisförmige Ausnehmung derart eingearbeitet, dass sich beim Zusammensetzen von Durchführungsflansch 11a und Isolierkörper 11b ein in der Draufsicht kreisförmiger Aufnahmeraum für einen Massepin 15 ergibt. Bei der Ausführung nach Fig. 2B hingegen ist der Aufnahmeraum für den Massepin 15 ausschließlich im Durchführungs-Flansch 11a eingearbeitet, so dass der Massepin beabstanded vom Isolierkörper 11b platziert ist.

Die Ausführung nach Fig. 2A kann grundsätzlich einen keramischen Isolierkörper 11b oder einen partiell aus Kunststoff gefertigten Isolierkörper aufweisen. Im ersteren Falle kann der Massepin 15 vor dem Sintern des Isolierkörpers 11b in dessen halbkreisförmige Ausnehmung eingefügt und durch den Sintervorgang form- und kraftschlüssig mit dem Isolierkörper verbunden werden. Er wird dann zusammen mit dem Isolierkörper durch an sich bekannte Prozesse in den Durchführungs-Flansch 11a eingebunden.

Sofern der Isolierkörper jedoch mindestens in demjenigen Abschnitt, wo der Massepin 15 angeordnet ist, aus einem Kunststoffmaterial besteht, kann der Massepin 15 an die entsprechende Stelle der Spritzgussform eingelegt und mit dem Kunststoffmaterial umspritzt werden, so dass er auch in diesem Falle mit dem Isolierkörper eine zusammenhängende Baueinheit bildet und diese in einem nachfolgenden Schritt in den Durchführungs-Flansch 11a eingebunden werden kann.

Bei der Konfiguration nach Fig. 2B sind (unter anderem) folgende Prozessabläufe möglich:
Bei einem Metall-Spritzgießverfahren (MiM): Der Massestift wird in die Spritzgußform des Flansches eingelegt und mit dem Flanschwerkstoff umspritzt. Bevorzugt soll der Werkstoff Titan als Flanschmaterial und Niob als Stiftmaterial eingesetzt werden. Aufgrund des höheren thermischen Wärmeausdehnungskoeffizienten des Titans findet ein Aufschrumpfen des Flansches auf den Niobstift beim Abkühlen nach dem Spritzgießen statt.

Alternativ kann ein Niobstift auch in einen additiv/generativ gedruckten Titanflansch vor dem Sintern eingelegt werden (in den Grünling oder Braunling). Durch das Entbindern/Sintern wird anschließend ein Formschluss des Stifts zum Flansch ausgebildet.

Weiter alternativ ist das Einbringen des Massestifts nach der Herstellung eines vorgefertigten (beispielsweise gefrästen) Flansches 11a möglich: Durch das Aufheizen des Ti-Flansches auf Ts>T>1200°C und das Einsetzen des Nb-Stifts in die vorgefertigte Öffnung des heißen Durchführungs-Flansches wird für einen Formschluss gesorgt, der auch die Wiederaufheizung durch den HTL Prozess übersteht.

Im Übrigen ist die Ausführung der Erfindung auch in einer Vielzahl von Abwandlungen der hier gezeigten Beispiele und weiter oben hervorgehobenen Aspekte der Erfindung möglich.

## Patentansprüche

1. Durchführung (11) eines, insbesondere implantierbaren, medizinelektronischen Gerätes (1) mit einem Gehäuse (3) und mindestens einem in dem Gehäuse aufgenommenen elektrischen oder elektronischen Bauelement (7), wobei die Durchführung einen Durchführungs-Flansch (11a) zum Verschluss einer Öffnung des Gehäuses und zur Halterung mindestens eines Anschlusselementes (13), das zum gehäuse-externen Anschluss des oder mindestens eines Bauelementes dient, in einem das Anschlusselement umgebenden Isolierelement (11b) aufweist und ein Massepin (15) in mechanischem und elektrischem Kontakt mit dem Durchführungs-Flansch zur Realisierung eines Masseanschlusses des Gerätes vorgesehen ist,
wobei der Massepin ausschließlich durch Form- und Kraftschluss in dem Durchführungs-Flansch oder dem Isolierelement, unter Berührung des Durchführungs-Flansches, fixiert ist.

2. Durchführung nach Anspruch 1, wobei das Isolierelement (11b) als massiver keramischer Isolierkörper ausgebildet und der Massepin (15) in den Isolierkörper eingesintert ist.

3. Durchführung nach Anspruch 1, wobei der Durchführungs-Flansch (11a) oder das Isolierelement (11b) ein Kunststoff-Spritzgussteil aufweist und der Massepin (15) mit dem Kunststoff-Spritzgussteil umspritzt ist.

4. Durchführung nach Anspruch 1, wobei der Durchführungs-Flansch (11a) einen Flanschkörper aus einem ersten Material aufweist und der Massepin (15) aus einem zweiten Material gebildet ist, das einen kleineren thermischen Ausdehnungskoeffizienten als das erste Material aufweist, und der Massepin in den Flanschkörper eingeschrumpft ist.

5. Durchführung nach Anspruch 4, wobei der Flanschkörper (11a) ein Metallkörper aus einem ersten Metall, insbesondere Titan, ist und der Massepin (15) aus einem zweiten Metall besteht, das einen kleineren thermischen Ausdehnungskoeffizienten als das erste Metall hat, insbesondere aus Niob.

6. Durchführung nach Anspruch 5, wobei der Massepin (15) durch Umspritzen mit dem ersten Metall in den Flanschkörper (11a) eingebettet ist.

7. Durchführung nach Anspruch 5, wobei der Massepin (15) durch thermisches Aufschrumpfen des Flanschkörpers (11a) auf den Massepin in den Flanschkörper eingebettet ist.

8. Durchführung nach Anspruch 7, wobei der Massepin (15) durch thermisches Aufschrumpfen eines additiv hergestellten Flanschkörpers auf den Massepin in den Flanschkörper (11a) eingebettet ist.

9. Verfahren zur Herstellung einer Durchführung (11) nach einem der vorangehenden Ansprüche, wobei der Massepin (15) in einer thermischen Prozessschrittfolge, die ein Erwärmen mindestens eines Materials des Durchführungs-Flansches (11a) oder Isolierelementes (11b), mit gleichzeitigem oder an das Erwärmen anschließendem Einbringen des Massepins direkt in das Material des Durchführungs-Flansches oder Isolierelementes bzw. in eine dort vorab ausgebildete Öffnung und das anschließende Abkühlen des Materials mit darin angeordnetem Massepin umfasst, fixiert wird.

10. Verfahren nach Anspruch 9, wobei das Isolierelement (11b) als massiver keramischer Isolierkörper ausgebildet ist und eine an die Gestalt des Massepins (15) angepasste Ausnehmung aufweist, der Massepin im grünen Zustand des Isolierkörpers in diesen eingefügt wird und der Isolierkörper mit eingefügtem Massepin in einem Sinterverfahren fertiggestellt wird.

11. Verfahren nach Anspruch 9, wobei der Massepin (15) in einen additiv/generativ gefertigten Durchführungs-Flansch (11a) vor dem Entbindern oder Sintern in eine angepasste Ausnehmung/Öffnung eingefügt wird und der Durchführungs-Flansch mit dem eingefügtem Massepin in einem Sinterverfahren fertiggestellt wird.

12. Verfahren nach Anspruch 9, wobei der Massepin (15) in eine Spritzgussform zur Bildung des Durchführungs-Flansches (11a) oder Isolierelementes (11b) eingelegt und mit einem in die Form eingebrachten Kunststoffmaterial umspritzt wird.

13. Verfahren nach Anspruch 9, wobei der Massepin (15) in eine Metallspritzform zur Bildung des Durchführungs-Flansches (11a) eingelegt und anschließend die Form mit einem verflüssigten Metall gefüllt und hierbei der Massepin mit dem Metall umspritzt wird.

14. Verfahren nach Anspruch 9, wobei ein vorgefertigter Durchführungs-Flansch (11a) aus einem ersten Metall, in dem eine Ausnehmung zur Aufnahme des Massepins (15) gebildet ist, erwärmt und im erwärmten Zustand der Massepin in die Ausnehmung eingebracht und anschließend der Durchführungs-Flansch mit eingebrachtem Massepin abgekühlt wird, derart, dass der Massepin in den Durchführungs-Flansch eingeschrumpft wird.

15. Medizinelektronisches Gerät (1) mit einer Durchführung (11) nach einem der Ansprüche 1 bis 8, insbesondere ausgebildet als Herzschrittmacher, Kardioverter oder Cochlear-Implantat.
